# EUROPÄISCHE PATENTSCHRIFT

(11) **EP 0 860 152 B1**
(45) Veröffentlichungstag und Bekanntmachung des Hinweises auf die Patenterteilung: **28.12.2005**
(21) Anmeldenummer: 98102930.9
(22) Anmeldetag: 19.02.1998
(51) Int. Cl.: A61F 2/44

(54) **Wirbelsäulenimplantat**
Spinal implant
Implant vertébral

(30) Priorität: 20.02.1997 DE 29703043 U
(43) Veröffentlichungstag der Anmeldung: 26.08.1998
(73) Patentinhaber: Signus Medizintechnik GmbH, 63755 Alzenau (DE)
(72) Erfinder: Siedler, Uwe, 63755 Alzenau (DE); Al Hami, Samir, Dr., 36043 Fulda (DE)
(74) Vertreter: Leinweber & Zimmermann

(56) Entgegenhaltungen:
- WO-A-96/40015
- FR-A- 2 703 580
- US-A- 4 834 757
- US-A- 5 306 309

## Beschreibung

Die Erfindung betrifft ein Wirbelsäulenimplantat zum Einsetzen zwischen zwei Wirbel,
mit einem ersten und einem zweiten auf in eingesetztem Zustand den Wirbeln zugewandten Verankerungsseiten angeordneten, im Querschnitt sägezahnförmigen Verankerungselement zur Verankerung des Implantats an den Wirbeln, wobei die Verankerungselemente im Querschnitt jeweils eine senkrecht auf der Verankerungsseite stehende erste Flanke und eine mit der ersten Flanke eine Spitze bildende zweite Flanke aufweisen, und
mit mindestens einem Durchbruch, der dazu ausgelegt ist, Knochensubstanz aufzunehmen.

Ein Wirbelsäulenimplantat der oben beschriebenen Art ist aus der DE 43 27 054 C1 bekannt. Wie in der DE 43 27 054 C1 ausgeführt, weisen die einzelnen Wirbel der Wirbelsäule unter anderem einen Wirbelkörper, einen Wirbelbogen, einen Dornfortsatz, zwei Querfortsätze und zwei obere sowie zwei untere Gelenkfortsätze auf. Die Wirbel sind über an ihren Wirbelkörpern (Corpus vertebrae) anliegende Zwischenwirbelscheiben (Disci intervertebralis) miteinander verbunden. Diese Zwischenwirbelscheiben bestehen aus flüssigkeitsreichem Faserknorpel und verbinden die einzelnen Wirbelkörper miteinander. Die Größe der Zwischenwirbelscheiben nimmt von oben nach unten entsprechend der im menschlichen Körper auftretenden Belastung zu. Die Zwischenwirbelscheiben dienen als elastische Puffer und dämpfen federnd Stöße ab. Es ist bekannt, daß sich die Zwischenwirbelscheiben verlagern können und daß der innere Gallertkern (Nucleus polposus) durch Risse im bindegewebsartigen knorpeligen äußeren Ring (Annulus fibrosus) austreten kann. Dabei kann die Zwischenwirbelscheibe teilweise in die Zwischenwirbellöcher (Foramina intervertebralia) bzw. in den Spinalkanal eintreten. Außerdem kann dieser Prolaps medial bzw. dorsal medial oder lateral sein. Derartige Prolapse treten am häufigsten an den L₄-L₅-, L₅-S₁- und C₆-C₇-Vertebrales auf. Werden derartige Prolapse nicht therapiert, kommt es zu irreversiblen Druckschädigungen von Nervenwurzeln oder zu Querschnittsläsionen. Sollte eine symptomatische Physiotherapie, z. B. Krankengymnastik oder Massage nicht hilfreich sein, muß die Zwischenwirbelscheibe (Diskus intervertebralis) operativ entfernt werden. Nun besteht die Möglichkeit der Implantation einer künstlichen Zwischenwirbelscheibe oder der Osteosynthese der beiden Wirbel über ein starres Wirbelsäulenimplantat, wie es eingangs beschrieben ist.

Das Wirbelsäulenimplantat nach der DE 43 27 054 C1 sieht zur Verankerung an den Wirbeln neben den Verankerungselementen noch Ankerungsbolzen vor, die in situ, d. h. nach dem Implantieren ausgefahren werden, um eine sichere Verankerung des Implantats an den Wirbeln zu gewährleisten. Die genannten Verankerungsbolzen machen das Implantat sehr kompliziert. Darüber hinaus müssen sie zur Verankerung ausgefahren werden, was den operativen Eingriff zum Implantieren erheblich verlängert.

Der Erfindung liegt daher die Aufgabe zugrunde, ein Wirbelsäulenimplantat anzugeben, das ohne die genannten Verankerungsbolzen auskommt.

Erfindungsgemäß wird die gestellte Aufgabe durch ein Wirbelsäulenimplantat nach Ansprüch 1 gelöst.

Dabei liegt der Erfindung die folgende Erkenntnis zugrunde: Zwar lehrt die DE 43 27 054 C1, daß die Sägezähne der Verankerungselemente derart ausgerichtet sind, daß sie in einander entgegengesetze Richtungen wirken, wie etwa ventral und dorsal; dadurch wird aber eine Verschiebesicherheit nur in den genannten Richtungen also dorsal und ventral erzielt. Verschiebungen beispielsweise in laterale Richtungen sind nicht unterbunden. Wenn nun erfindungsgemäß mindestens zwei Verankerungselemente vorgesehen sind, bei denen die normalen auf die "senkrechten" Flanken weder parallel noch antiparallel zueinander liegen, so wird dadurch eine Sicherung gegen Verschiebung in alle Richtungen, also dorsal/ventral und lateral erzielt.

Wegen dieser Verschiebesicherung sind zusätzliche Ankerbolzen entbehrlich. Vielmehr muß das Implantat nur noch zwischen die Wirbel gesetzt werden. Die von den erfindungsgemäßen Verankerungselementen herrührende Verschiebesicherheit reicht alleine aus.

Prinzipiell können die genannten Normalen jeden beliebigen Winkel miteinander einschließen, solange dieser Winkel nicht 180 oder 360 ° beträgt. Erfindungsgemäß bevorzugt schließen sie jedoch einen Winkel von 90 ° ein.

Das Wirbelsäulenimplantat kann prinzipiell jede beliebige Form haben. Bevorzugt hat es jedoch zwei weitere Seiten, die den Verankerungsseiten und einander benachbart sind, wobei die ersten Flanken der beiden Verankerungselemente jeweils mit einer der beiden weiteren Seiten fluchten. Dadurch gibt es in der Nachbarschaft der zur Verschiebesicherung dienenden "senkrechten" Flanken keine quer dazu liegenden Absätze oder Flächen, die eine zuverlässige Verankerung beeinträchtigen könnten.

Weiter bevorzugt weist das Wirbelsäulenimplantat nach der Erfindung ein viertes Verankerungselement mit der gleichen Kontur wie das zweite Verankerungselement auf, wobei die Normale auf die erste Flanke des vierten Verankerungselements antiparallel zu der Normalen auf die erste Flanke des zweiten Verankerungselements liegt. Mit anderen Worten ist erfindungsgemäß zusätzlich zu dem zweiten Verankerungselement ein Verankerungselement mit antiparalleler Ausrichtung vorgesehen. Dadurch wird die Verankerung gegen dorsale, ventrale und laterale Verschiebungen weiter verbessert.

Sowohl für das Implantieren als auch für die Sicherung gegen Verschiebungen hilfreich ist es, wenn die zweite Flanke des ersten, zweiten, dritten und/oder vierten Verankerungselements im Querschnitt konkav bogenförmig ist.

Bevorzugt haben am Rande liegende Verankerungselemente die Kontur eines längs einer Vertikalebene halbierten Zeltes.

Zusätzlich können weitere, im Zentrum einer jeden Verankerungsseite liegende Verankerungselemente vorgesehen sein, die die Kontur eines (nicht halbierten) Zeltes haben, mit im Querschnitt bogenförmig auf die Spitze zulaufenden Flanken.

Zur weiteren Steigerung der Zuverlässigkeit der Sicherung gegen Verschieben sind erfindungsgemäß bevorzugt mehrere Verankerungselemente in einer eine Verankerungsseite am Rand kronenartig umlaufenden Reihe vorgesehen.

Der Durchbruch kann erfindungsgemäß von der einen zu der anderen Verankerungsseite verlaufen.

Alternativ oder zusätzlich kann aber auch vorgesehen sein, daß ein Durchbruch quer zu einer senkrecht auf einer der Verankerungsseiten stehenden Achse verläuft.

Insbesondere dann, wenn Durchbrüche beider der vorgenannten Arten vorgesehen sind, kann von den Durchbrüchen aufgenommene Knochensubstanz zur weiteren Verankerung dienen.

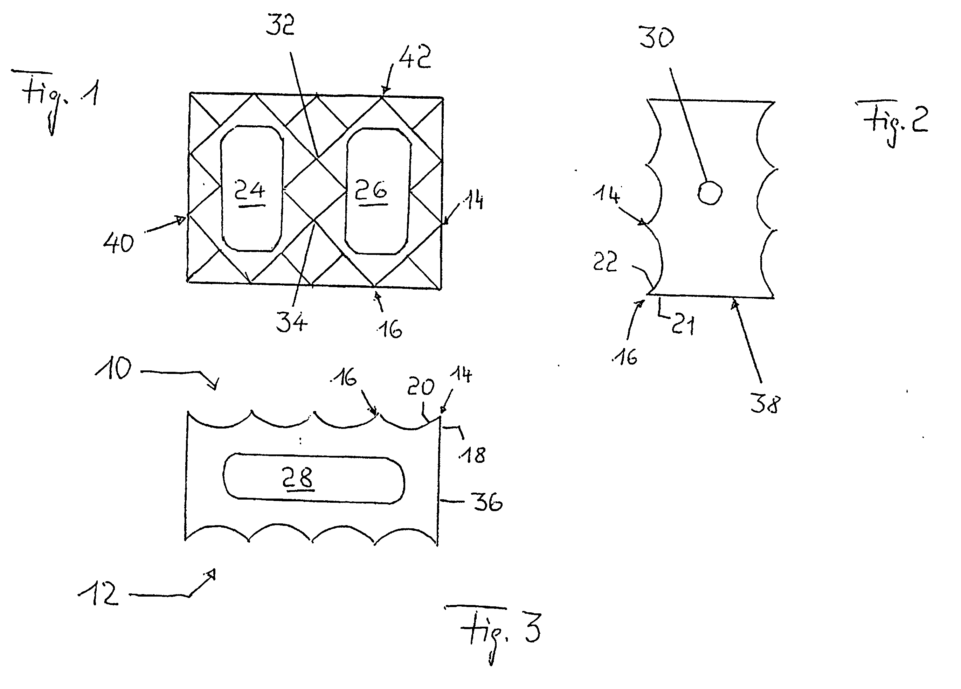

Nachstehend ist die Erfindung anhand eines bevorzugten Ausführungsbeispiels unter Bezugnahme auf die beiliegende Zeichnung mit weiteren Einzelheiten näher erläutert. Dabei zeigen
- Fig. 1: die Draufsicht des Implantats,
- Fig. 2: eine Umklappung nach rechts und
- Fig. 3: eine Umklappung nach unten.

Das in der Zeichnung gezeigte Ausführungsbeispiel ist quaderförmig. Es hat zwei Verankerungsseiten 10 und 12, die in eingesetztem Zustand den (nicht gezeigten) Wirbeln zugewandt sind.

Auf den beiden Verankerungsseiten 10 und 12 sind Verankerungselemente angeordnet, von denen diejenigen im folgenden näher erläutert sind, die mit den Bezugszahlen 14 und 16 bezeichnet sind. Das Verankerungselement 14 hat eine senkrecht auf der Verankerungsseite 10 stehende erste Flanke 18 und eine mit der ersten Flanke eine Spitze bildende zweite Flanke 20. Ebenso hat das Verankerungselement 16 eine senkrecht auf der Verankerungsseite 10 stehende erste Flanke 21 und eine mit der ersten Flanke eine Spitze bildende zweite Flanke 22. Die beiden genannten ersten Flanken 18 und 21 schließen miteinander einen rechten Winkel ein. Da insbesondere die ersten Flanken 21 und 18 eine Sicherheit gegen Verschieben in ventraler/dorsaler Richtung bzw. in lateraler Richtung geben, sind keine weiteren Verankerungseinrichtungen erforderlich.

In dem gezeigten Ausführungsbeispiel des erfindungsgemäßen Wirbelsäulenimplantats sind Durchbrüche 24, 26 und 28 zum Aufnehmen von Knochensubstanz vorgesehen. Dadurch ist ein zuverlässiges Einbinden des Implantats möglich.

Auf der im eingesetzten Zustand ventral ausgerichteten Seite des in der Zeichnung gezeigten Ausführungsbeispiels ist eine Gewindebohrung 30 vorgesehen. Sie dient zur Aufnahme eines entsprechenden Werkzeugs des Chirurgen, mit dem er das Implantat beim Einsetzen führen kann.

Oben sind die Verankerungselemente 14 und 16 im einzelnen beschrieben worden. Von diesen Verankerungselementen, also solchen mit einer senkrecht auf der Verankerungsseite stehenden ersten Flanke sind aber darüber hinaus noch weitere Exemplare vorgesehen. Sie sind in Reihen angeordnet, welche die Verankerungsseiten 10 und 12 jeweils kronenartig umlaufen.

Daneben gibt es aber noch andere Verankerungselemente, nämlich beispielsweise die Verankerungselemente 32 und 34 in etwa im Zentrum einer jeden Verankerungsseite. Diese Verankerungselemente 32 und 34 sind in etwa zeltartig. Unter zeltartig wird in diesem Zusammenhang verstanden, daß sie - wie eine Pyramide - spitz zulaufen, sich aber von der Pyramide dadurch unterscheiden, daß sie im Querschnitt gesehen bogenförmig auf die Spitze zulaufende Flanken haben.

Dementsprechend haben die am Rand liegenden Verankerungselemente 14 und 16 jeweils die Kontur eines halben Zeltes. Bei ihnen fluchtet die durch die Halbierung entstehende senkrecht auf der Verankerungsseite stehende erste Flanke jeweils mit einer Begrenzungsseite des Implantats.

Weitere Verankerungselemente mit der Kontur eines "halben Zeltes" sind mit den Bezugszahlen 40 bzw. 42 bezeichnet. Ihre "senkrechten" ersten Flanken liegen antiparallel zu den "senkrechten" ersten Flanken der Verankerungselemente 14 und 16.

Weitere Seiten des quaderförmigen Implantats sind mit den Bezugszahlen 36 bzw. 38 bezeichnet.

Die in der vorstehenden Beschreibung, den Ansprüchen sowie der Zeichnung offenbarten Merkmale der Erfindung können sowohl einzeln als auch in beliebigen Kombinationen für die Verwirklichung der Erfindung in ihren verschiedenen Ausführungsformen wesentlich sein.

## Patentansprüche

1. Wirbelsäulenimplantat zum Einsetzen zwischen zwei Wirbel,
mit einem ersten (14) und einem zweiten (16) auf in eingesetztem Zustand den Wirbeln zugewandten Verankerungsseiten (10, 12) angeordneten, im Querschnitt sägezahnförmigen Verankerungselement zur Verankerung des Implantats an den Wirbeln, wobei die Verankerungselemente (14, 16) im Querschnitt jeweils eine senkrecht auf der Verankerungsseite (10, 12) stehende erste Flanke (18, 21) und eine mit der ersten Flanke (18, 21) eine Spitze bildende zweite Flanke (20, 22) aufweisen, und
mit mindestens einem Durchbruch (24, 26, 28), der dazu ausgelegt ist, Knochensubstanz aufzunehmen,
wobei
die Normalen auf die ersten Flanken (18, 21) der beiden Verankerungselemente (14, 16) weder parallel noch antiparallel zueinander liegen, und daß ein drittes Verankerungselement (40) mit der gleichen Kontur wie das erste Verankerungselement (14) vorgesehen ist, wobei die Normale auf die erste Flanke des dritten Verankerungselements (40) antiparallel zu der Normalen auf die erste Flanke des ersten Verankerungselements (14) liegt.

2. Wirbelsäulenimplantat nach Anspruch 1, **dadurch gekennzeichnet, daß** die Normalen des ersten und zweiten Verankerungselements einen Winkel von 90 ° einschließen.

3. Wirbelsäulenimplantat nach Anspruch 1 oder 2, **dadurch gekennzeichnet, daß** es zwei weitere Seiten (36, 38) hat, die den Verankerungsseiten (10, 12) und einander benachbart sind, wobei die ersten Flanken (18, 21) des ersten und zweiten Verankerungselements (14, 16) jeweils mit einer der beiden weiteren Seiten (36, 38) fluchten.

4. Wirbelsäulenimplantat nach einem der vorangehenden Ansprüche, **gekennzeichnet durch** ein viertes Verankerungselement (42) mit der gleichen Kontur wie das zweite Verankerungselement (16), wobei die Normale auf die erste Flanke des vierten Verankerungselements (42) antiparallel zu der Normalen auf die erste Flanke des zweiten Verankerungselements (16) liegt.

5. Wirbelsäulenimplantat nach einem der vorangehenden Ansprüche, **dadurch gekennzeichnet, daß** die zweite Flanke (20, 22) des ersten, zweiten, dritten und/oder vierten Verankerungselements (14, 16, 40, 42) im Querschnitt konkav bogenförmig ist.

6. Wirbelsäulenimplantat nach einem der vorangehenden Ansprüche, **dadurch gekennzeichnet, daß** am Rande liegende Verankerungselemente (14, 16, 40, 42) die Kontur eines längs einer Vertikalebene halbierten Zeltes haben.

7. Wirbelsäulenimplantat nach einem der vorangehenden Ansprüche, **gekennzeichnet durch** weitere, im Zentrum einer jeden Verankerungsseite (10,12) liegende Verankerungselemente (32, 34) mit der Kontur eines Zeltes, mit im Querschnitt bogenförmig auf die Spitze zulaufenden Flanken.

8. Wirbelsäulenimplantat nach einem der vorangehenden Ansprüche, **dadurch gekennzeichnet, daß** mehrere Verankerungselemente (14, 16, 40, 42) in einer eine Verankerungsseite (10, 12) am Rand kronenartig umlaufenden Reihe vorgesehen sind.

9. Wirbelsäulenimplantat nach einem der vorangehenden Ansprüche, **dadurch gekennzeichnet, daß** der Durchbruch (24, 26) von der einen zu der anderen Verankerungsseite (10, 12) verläuft.

10. Wirbelsäulenimplantat nach einem der vorangehenden Ansprüche, **dadurch gekennzeichnet, daß** der Durchbruch (28) quer zu einer senkrecht auf einer der Verankerungsseiten (10, 12) stehenden Achse verläuft.

## Claims

1. Spinal column implant for insertion between two vertebrae,
with a first anchoring element (14) and a second anchoring element (16) for anchoring the implant to the vertebrae, which anchoring elements are sawtooth-shaped in cross-section and, in the inserted state, are arranged on anchoring sides (10, 12) facing the vertebrae, the anchoring elements (14, 16) each having in cross-section a first flank (18, 21) perpendicular to the anchoring side (10, 12), and a second flank (20, 22) forming a point with the first flank (18, 21), and
with at least one through-passage (24, 26, 28) which is designed to receive bone substance,
where the normals to the first flanks (18, 21) of the two anchoring elements (14, 16) lie neither parallel nor antiparallel to one another, and a third anchoring element (40) with the same contour as the first anchoring element (14) is provided, where the normal to the first flank of the third anchoring element (40) lies antiparallel to the normal to the first flank of the first anchoring element (14).

2. Spinal column implant according to Claim 1, **characterized in that** the normals of the first and second anchoring element enclose an angle of 90°.

3. Spinal column implant according to Claim 1 or 2, **characterized in that** it has two further sides (36, 38) which are adjacent to the anchoring sides (10, 12) and to one another, the first flanks (18, 21) of the first and second anchoring element (14, 16) each being flush with one of the two other sides (36, 38).

4. Spinal column implant according to one of the preceding claims, **characterized by** a fourth anchoring element (42) with the same contour as the second anchoring element (16), where the normal to the first flank of the fourth anchoring element (42) lies antiparallel to the normal to the first flank of the second anchoring element (16).

5. Spinal column implant according to one of the preceding claims, **characterized in that** the second flank (20, 22) of the first, second, third and/or fourth anchoring element (14, 16, 40, 42) is curved concave in cross-section.

6. Spinal column implant according to one of the preceding claims, **characterized in that** anchoring elements (14, 16, 40, 42) lying at the edge have the contour of a tent halved along a vertical plane.

7. Spinal column implant according to one of the preceding claims, **characterized by** further anchoring elements (32, 34) with the contour of a tent which lie at the centre of each anchoring side (10, 12) and have flanks tapering, arcuately in cross-section, towards the point.

8. Spinal column implant according to one of the preceding claims, **characterized in that** a plurality of anchoring elements (14, 16, 40, 42) are provided in a row running in a crown shape round the edge of an anchoring side (10, 12).

9. Spinal column implant according to one of the preceding claims, **characterized in that** the through-passage (24, 26) runs from one anchoring side (10, 12) to the other.

10. Spinal column implant according to one of the preceding claims, **characterized in that** the through-passage (28) runs transverse to an axis perpendicular to one of the anchoring sides (10, 12).

## Revendications

1. Implant vertébral pour l'insertion entre deux vertèbres, avec un premier (14) et un second (16) élément d'ancrage en forme de dents de scie en section transversale, disposés sur des côtés d'ancrage (10,12) orientés à l'état inséré vers les vertèbres, pour l'ancrage de l'implant aux vertèbres, où les éléments d'ancrage (14,16) présentent en section transversale à chaque fois un premier flanc (18,21) s'étendant perpendiculairement au côté d'ancrage (10,12) et un second flanc (20,22) formant une pointe avec le premier flanc (18,21), et
avec au moins un perçage (24, 26, 28) qui est conçu pour recevoir de la substance osseuse,
où
les normales aux premiers flancs (18, 21) des deux éléments d'ancrage (14, 16) ne sont ni parallèles ni antiparallèles l'une à l'autre, et en ce qu'un troisième élément d'ancrage (40) ayant le même contour que le premier élément d'ancrage (14) est prévu, où la normale au premier flanc du troisième élément d'ancrage (40) est antiparallèle à la normale au premier flanc du premier élément d'ancrage (14).

2. Implant vertébral selon la revendication 1, **caractérisé en ce que** les normales des premier et second éléments d'ancrage forment un angle de 90°.

3. Implant vertébral selon la revendication 1 ou 2, **caractérisé en ce qu'**il possède deux autres côtés (36, 38) qui avoisinent les côtés d'ancrage (10, 12) et l'un l'autre, où les premiers flancs (18, 21) des premier et second éléments d'ancrage (14, 16) sont à chaque fois alignés avec l'un des deux autres côtés (36, 38).

4. Implant vertébral selon l'une des revendications précédentes, **caractérisé par** un quatrième élément d'ancrage (42) ayant le même contour que le deuxième élément d'ancrage (16), où la normale au premier flanc du quatrième élément d'ancrage (42) est antiparallèle à la normale au premier flanc du second élément d'ancrage (16).

5. Implant vertébral selon l'une des revendications précédentes, **caractérisé en ce que** le second flanc (20, 22) du premier, deuxième, troisième et/ou quatrième élément d'ancrage (14, 16, 40, 42) présente une courbure concave en section transversale.

6. Implant vertébral selon l'une des revendications précédentes, **caractérisé en ce que** des éléments d'ancrage (14, 16, 40, 42) situés au bord ont le contour d'une tente séparé en deux le long d'un plan vertical.

7. Implant vertébral selon l'une des revendications précédentes, **caractérisé par** d'autres éléments d'ancrage (32, 34) situés au centre de chaque côté d'ancrage (10, 12) ayant le contour d'une tente, avec des flancs s'étendant en section transversale en forme d'arc vers la pointe.

8. Implant vertébral selon l'une des revendications précédentes, **caractérisé en ce que** plusieurs éléments d'ancrage (14, 16, 40, 42) sont prévus en une rangée entourant en forme de couronne un côté d'ancrage (10, 12) au bord.

9. Implant vertébral selon l'une des revendications précédentes, **caractérisé en ce que** le perçage (24, 26) s'étend d'un côté d'ancrage (10, 12) vers l'autre.

10. Implant vertébral selon l'une des revendications précédentes, **caractérisé en ce que** le perçage (28) s'étend transversalement à un axe s'étendant perpendiculairement à l'un des côtés d'ancrage (10, 12).
